(19) **Europäisches Patentamt European Patent Office Office européen des brevets**

(11) **EP 3 412 115 B1**

(12) **EUROPEAN PATENT SPECIFICATION**

(45) Date of publication and mention of the grant of the patent:
**24.11.2021 Bulletin 2021/47**

(21) Application number: **16895683.7**

(22) Date of filing: **24.03.2016**

(51) Int Cl.:
*H05B 6/70* (2006.01)        *H05B 6/80* (2006.01)
*H05B 6/78* (2006.01)        *A23L 3/01* (2006.01)
*A61L 2/12* (2006.01)

(86) International application number:
**PCT/US2016/024025**

(87) International publication number:
**WO 2017/164879 (28.09.2017 Gazette 2017/39)**

(54) **MULTI-ZONE PROCESSING SYSTEM FOR APPLYING ELECTROMAGNETIC ENERGY**

MEHRZONIGES VERARBEITUNGSSYSTEM ZUM ANWENDEN VON ELEKTROMAGNETISCHER ENERGIE

SYSTÈME DE TRAITEMENT MULTIZONE POUR L'APPLICATION D'ÉNERGIE ÉLECTROMAGNÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**12.12.2018 Bulletin 2018/50**

(73) Proprietor: **HBC Holding Company, LLC Raleigh, NC 27601 (US)**

(72) Inventors:
• **DRUGA, Michael Conley Raleigh, NC 27614 (US)**
• **CORONEL, Pablo Marcelo Apex, NC 27502 (US)**

• **CHAPMAN, Steven Lawrence Holly Spring, NC 27540 (US)**
• **KENNER, Thomas Henry Asheboro, NC 27205 (US)**

(74) Representative: **Yeadon IP Limited Nexus Discovery Way Leeds LS2 3AA (GB)**

(56) References cited:
**WO-A1-2015/196218        JP-B2- 4 603 932
US-A1- 2005 082 283        US-A1- 2005 127 068
US-A1- 2007 131 678        US-A1- 2011 132 902
US-A1- 2011 290 789        US-A1- 2013 122 160
US-A1- 2013 122 160        US-A1- 2016 050 722**

**Description**

BACKGROUND

It is often beneficial to process materials by heating them. One technique for heating material is to boil it in a can. Boiling has many drawbacks. One drawback is very low efficiency. Boiling processes can have efficiencies as low as 10 to 20%. Another drawback is that the material is not heated uniformly. Rather, heat is applied from outside of the can, resulting in a cold center and hot perimeter. Heat conducts inward through the can and then through the outermost material until reaching the material in the center. As a result, uniform heating cannot be achieved. Some materials are damaged by such uneven heating, and overcooking of the outermost material is typically required in order to adequately heat the innermost material.

Heating materials using electromagnetic energy, such as microwave energy, can be much more efficient than other forms of heating and can result in much less damage to the materials, and thus higher quality products.

US 2013/0122160 A1 describes a system for processing materials. Flowing heterogeneous, multiphase foods and biomaterials are exposed to single or multiple stages of electromagnetic energy under continuous flow conditions within conduits passing through electromagnetic exposure chambers.

US 2007/0131678 A1 describes heating and drying devices including generally rectangular waveguide applicators forming exposure chambers for uniformly heating materials.

US 2005/0082283 A1 describes a microwave dryer which may be used to dry materials such as a sewage sludge.

JP 4603932 B2 describes a heating device for heating foods.

US 2011/0132902 A1 describes a microwave-based liquid vaporization system.

SUMMARY

There is provided a material processing system according to claim 1. In general terms, this disclosure is directed to a system for applying electromagnetic energy. In one possible configuration and by non-limiting example, a multi-zone processing system is disclosed for applying the electromagnetic energy. In some embodiments the multi-zone processing system includes a multi-zone applicator. Various aspects are described in this disclosure, which include, but are not limited to, the following aspects.

One aspect is a material processing system comprising: an energy generating system comprising a plurality of electromagnetic energy generators; a pump configured to provide a flow of material to be processed; and a multi-zone applicator spaced from the energy generating system and comprising: a plurality of application zones, each of the application zones configured to receive electromagnetic energy generated at least one of the plurality of generators; a material conduit coupled to the pump to receive the flow of material and extending through the application zones, at least part of the material conduit being transparent to the electromagnetic energy to allow the material to be exposed to the electromagnetic energy as the material passes through the application zones, wherein the application zones have the same shape, are arranged in a common generally horizontal orientation, and are vertically aligned, and wherein the material conduit has a continuously upward slope throughout the multi-zone applicator.

Further aspects include any one or more of the systems illustrated in the drawings.

Other aspects include any one or more of the methods illustrated in the drawings.

BRIEF DESCRIPTION OF THE DRAWINGS

FIG. 1 is a schematic block diagram illustrating an example material processing system.
FIG. 2 is a schematic block diagram illustrating an example energy generating system and a portion of an energy transfer assembly of the material processing system shown in FIG. 1.
FIG. 3 is a center cross-sectional view of another example portion of the energy transfer assembly.
FIG. 4 is an isometric side view of an example multi-zone applicator of the material processing system of FIG. 1.
FIG. 5 is another isometric side of the example multi-zone applicator shown in FIG. 4.
FIG. 6 is a perspective view of an example stand of the example multi-zone applicator of FIG. 4.
FIG. 7 is a top view of an example application zone.
FIG. 8 is a side view of the example application zone shown in FIG. 7.
FIG. 9 is another side view of the example application zone shown in FIG. 7.
FIG. 10 is another side view of the example application zone shown in FIG. 7.
FIG. 11 is a perspective view of an example splitter.
FIG. 12 is a schematic block diagram illustrating an example of a control system.
FIG. 13 is a screen shot illustrating an example graphical user interface of the control system shown in FIG. 12.
FIG. 14 is a flow chart illustrating an example method of controlling a material flow rate using the control system shown in FIG. 12.
FIG. 15 is a flow chart illustrating an example method of adjusting a pressure using the control system shown in FIG. 12.
FIG. 16 is a flow chart illustrating a method of operating a material processing system.

FIG. 17 is a flow chart illustrating an example method of controlling a material processing system.

FIG. 18 is a flow chart illustrating an example method of controlling a material processing system.

FIG. 19 illustrates a temperature profile having a linear ramp profile.

FIG. 20 illustrates a temperature profile having a ramp, soak, ramp profile.

FIG. 21 illustrates a temperature profile having a tapered profile.

DETAILED DESCRIPTION

Various embodiments will be described in detail with reference to the drawings, wherein like reference numerals represent like parts and assemblies throughout the several views. Reference to various embodiments does not limit the scope of the claims attached hereto. Additionally, any examples set forth in this specification are not intended to be limiting and merely set forth some of the many possible embodiments for the appended claims.

In the following description inches are occasionally used. One inch is equal to 2.54 centimetres.

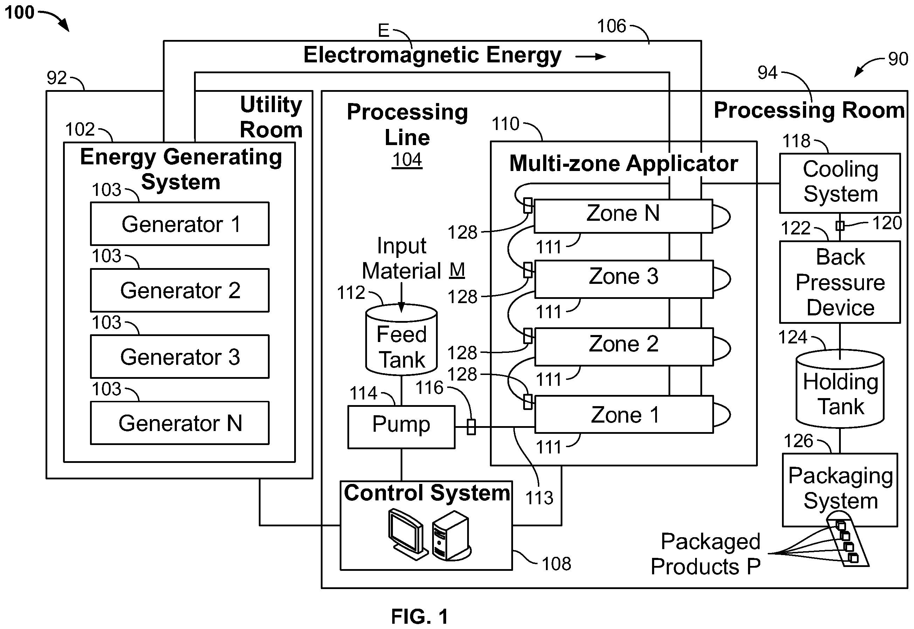

FIG. 1 is schematic block diagram illustrating an example material processing system 100 at an example material processing facility 90. The example material processing facility includes a utility room 92 and a processing room 94. The example material processing system 100 includes an energy generating system 102, a processing line 104, and an energy transfer assembly 106. The material processing system 100 operates to process materials M input into the system, and to output the final product P, such as a packaged product P.

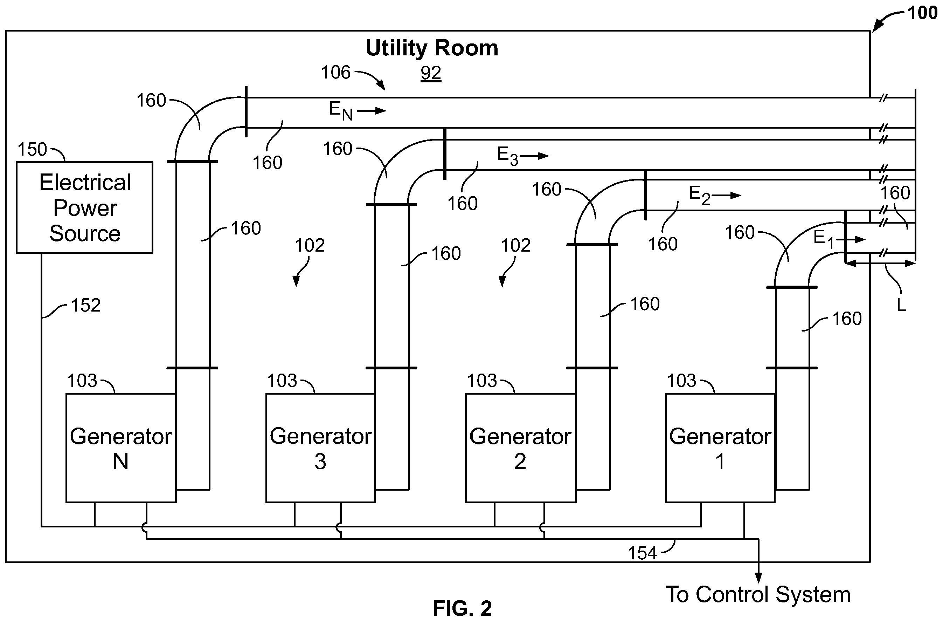

In some embodiments the material processing facility 90 is divided into multiple rooms or sections to allow compartmentalization of certain components of the material processing system 100. In this example, the material processing facility includes a utility room that houses the energy generating system 102, and allows the generators 103 (including generators 1, 2, 3, ... N) contained therein to be separated from the rest of the processing line, along with electrical power supplies and the like. An example of the energy generating system 102 is illustrated and described in more detail with reference to FIG. 2. Walls or dividers can be used to separate and compartmentalize components of the material processing system 100 within the facility 90, for example.

In some embodiments the processing line 104 is kept in a separate processing room 94 of the facility. In some embodiments the processing room 94 is a sterile area of the material processing facility 90 to reduce the chance of contamination of the material M as it is processed. Additional rooms or dividers can be used in other embodiments. Further, in some embodiments the rooms can be in separate buildings. Alternatively, the material processing system 100 can also be contained within a single room in another possible embodiment.

An energy transfer assembly 106 is provided to transfer the electromagnetic energy generated by the energy generating system 102 to the processing line 104 where it can be used for processing the material M. In some embodiments the energy generating system 102 is arranged a distance away from the processing room 94, and the energy transfer assembly 106 is arranged to transfer the energy between the spaced components as discussed in further detail with reference to FIG. 2.

The processing line 104 can have a variety of processing components, but in some embodiments includes at least a multi-zone applicator 110. Additional exemplary components illustrated in FIG. 1 include a feed tank 112, a pump 114, a flow rate sensor 116, a cooling system 118, a pressure sensor 120, a back pressure device 122, a holding tank 124, and a packaging system 126 for outputting the final packaged products P.

One or more feed tanks 112 are provided in some embodiments to receive and store input materials M prior to processing of those materials through the multi-zone applicator 110. Other input devices can also be used, such as an input pipe providing a continuous flow of input materials from another system or source.

A variety of materials M can be processed through the material processing system 100. Typically such materials will include at least some polar molecules or conducting ions so that the materials will react to exposure to the electromagnetic fields. The electromagnetic fields cause movement or rotation of the molecules and ions resulting in heating of the material. The material can include heterogeneous or homogeneous materials. Materials often include at least some liquid, but can include liquids and solids. Non-liquid materials can also be used when such materials are pumpable by the pump 114 and flowable through the material conduits. A liquid can also be added to materials to allow such materials to be processed by the material processing system 100. Examples of materials include food products (e.g., soup, juice, cranberry sauce, vegetables, mashed potatoes), other biomaterials, pharmaceuticals, chemicals, and waste products (e.g., bio-waste).

The processing line 104 includes a material conduit 113 that guides the flow of the material M through the processing line 104, such as from the feed tank 112 to the packaging system 126. The material conduit 113 can include multiple pieces or segments and is connected to or passes through various components of the processing line 104 as discussed herein. In some embodiments the material conduit 113 is a continuous length of conduit that supplies a continuous flow of material while the material processing system is applying electromagnetic energy to the material.

The pump 114 is coupled to the feed tank by the material conduit 113 to receive the material M from the feed tank 112 and pump the material into the multi-zone applicator 110. A positive displacement pump is typically used to ensure that the material M can only move in one direction through the system. In some embodiments the pump 114 operates to prevent back flow of the material. The pump 114 also provides a continuous, consistent flow rate. In some embodiments the flow rate is adjustable by the control system 108, such as based on the type of materials M being processed. In some embodiments the pump 114 is a progressive cavity pump or a piston pump.

Some embodiments include a flow rate sensor 116 arranged along the material conduit after the pump 114 and measures the flow rate of the material through the material conduit 113. In some embodiments the pump 114 provides a known flow rate such that the flow rate sensor 116 may not be required, however even in such cases the flow rate sensor 116 can be used to ensure a proper flow rate. In a continuous flow system the flow rate sensor 116 can be arranged anywhere along the length of the material conduit 113, or multiple flow rate sensors could be used.

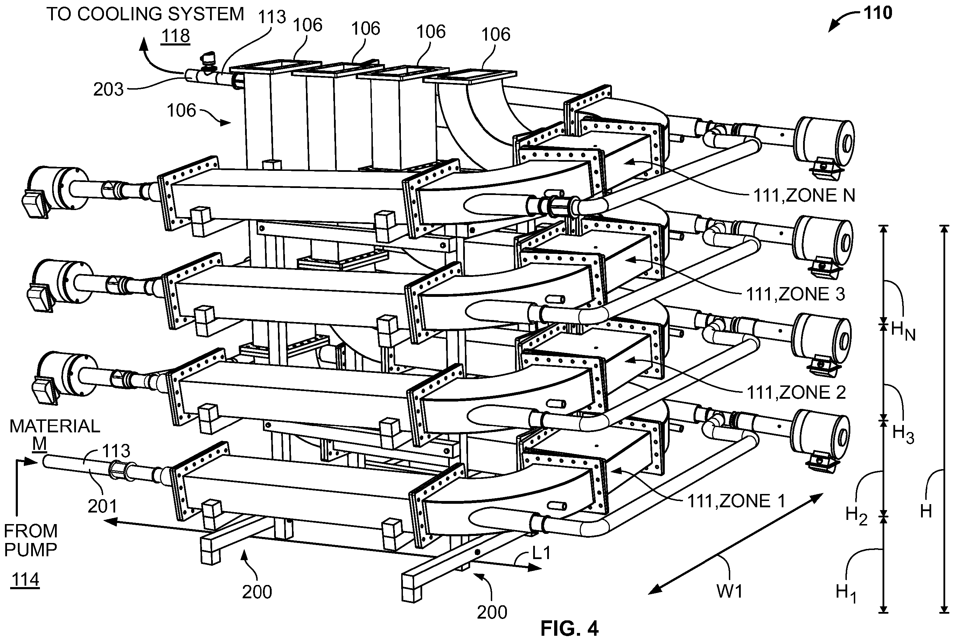

The multi-zone applicator 110 operates to receive the material M through the material conduit 113 and to expose the material to electromagnetic energy from the energy generating system 102. In this example the multi-zone applicator 110 includes a plurality of application zones 111 (including zones 1, 2, 3, ... N). The material conduit 113 passes through each of the application zones 111 where the energy from the generators 103 is delivered to the material. Temperature sensors 128 are provided and used in some embodiments to measure the temperature of the material before and after passing through each of the application zones 111. In some embodiments the multi-zone applicator 110 is an aseptic processor. Examples of the multi-zone applicator 110 are illustrated and described in further detail herein with reference to FIGS. 4-11.

A cooling system 118 is provided in some embodiments to receive and cool the material M after being processed by the multi-zone applicator 110. In some embodiments the cooling system 118 includes another temperature sensor to measure the temperature of the material M after cooling.

A pressure sensor 120 and back pressure device 122 are provided in some embodiments to measure and control the pressure in the material conduit 113. The back pressure device 122 can be used for example to increase the pressure above atmospheric pressure. One benefit is that by increasing the pressure, the boiling point of liquid in the material M is also increased allowing the material M to be heated to higher temperature without changing the phase of the liquid to gas. The option to obtain higher temperatures can improve the effectiveness of the processing. For example, to more effectively kill bacteria that may be present in a food product. However, other embodiments can operate at or near atmospheric pressure and therefore may not include a back pressure device 122 or pressure sensor 120. One example of a back pressure device is a valve operable to increase or decrease a resistance of the material flow through the material conduit 113. Another example of a back pressure device is a pump, such as a positive displacement pump, which can be connected to or coupled with the material conduit 113 and used to hold an appropriate back pressure within the material conduit 113.

Some embodiments include a holding tank 134 to temporarily store the material M after it has been processed. The holding tank 134 can be used, for example, to store the material M until it is packaged, to allow further cooling of the material M, for mixing of the material M, to allow time for chemical reactions or setting of products to occur, for temporary storage prior to advancing the material to a subsequent material processing system, or combinations of these. In other possible embodiments the material M can proceed directly to a packaging system 126 without a holding tank 124. The holding tank may alternatively be a final destination for the material M without then proceeding to a subsequent packaging system 126.

The packaging system 126 receives the material M and packages it to generate packaged products P. In some embodiments the packaging system 126 is a filler, such as an aseptic bag or box filler. Some embodiments include a vacuum sealer. The packaging system 126 can in other embodiments operate to fill other types of packaging, such as cartons, cans, buckets, drums, etc. After being inserted and sealed in the appropriate packaging, the packaged products P are output from the material processing system 100.

In this example the material processing system 100 also includes a control system 108. The control system 108 includes at least a processing device, and at least a computer-readable medium. The control system 108 in electrical or data communication with the energy generating system 102 and the processing line 104 to control the operation of the material processing system 100. The control system 108 can be located in the processing room, or the utility room, or at any other desired location including a location remote from the processing facility 90. The control system 108 can utilize any suitable electrical or data communication system, including direct electrical connection, connection through a data communication network, or a wireless data communication system including such as using a Wi-Fi or cellular data communication network.

In some embodiments the material processing facility 90 includes multiple material processing systems 100, energy generating systems 102, processing lines 104, energy transfer assemblies 106, and/or control systems 108.

FIG. 2 is a schematic block diagram illustrating an example of the energy generating system 102, and a portion of the energy transfer assembly 106. The energy generating system 102 can be located in a utility room 92 of a processing facility 90, such as shown in FIG. 1. In this example, the energy generating system 102 includes a plurality of generators 103 including, for example, generators 1, 2, 3, ... N, and an electrical power source 150.

The electrical power source 150 supplies electricity to power the generators 103. The power source 150 typically receives power from an external power source, such as from AC mains power. The power source 150 can include one or more terminal boxes, fuses or circuit breakers, and other possible power filtering or conditioning electronics. Electrical wiring 152 delivers the electrical power from the electrical power source 150 to the generators 103.

The generators 103 operate to generate electromagnetic energy E. In some embodiments, the generators 103 are microwave generators that generate microwave energy. The microwave energy is electromagnetic energy having a frequency in a range from 300 MHz to 300 GHz. Typically the microwave generators 103 generate microwave energy having a frequency of about 915 MHz or about 2.45 GHz, though other frequencies or combinations of frequencies can also be used in other embodiments.

In some embodiments the generators 103 are industrial microwave generators having maximum power output in a range from about 10 kilowatts to about 150 kilowatts. The inventors have found that the cost per watt of currently commercially available industrial microwave generators 103 is the lowest for 100 kilowatt generators, therefore in at least some embodiments the generators 103 are 100 kilowatt generators. Other embodiments can have other maximum power outputs. One example of a suitable generator 103 is the ATM 1010 Transmitter Assembly 100 kilowatt microwave generator available from Applied Microwave Technology, Inc. of Cedar Rapids, IA. Some embodiments generate microwave energy having a frequency of about 896, 915, or 922 MHz, and generate 75 or 100 kilowatts of power.

The generators 103 also typically have a variable power output, which can be adjusted and controlled by the control system 108, such as through the control wiring 154. The generators can be turned on and off by the control system 108, and the power output can also be adjusted by the control system between a minimum power output and the maximum power output (e.g., 25%, 50%, 75%, or 100%).

The example energy generating system 102 includes a plurality of generators 103. FIG. 2 illustrates four generators 103, including generator 1, 2, 3, and N. The variable N represents any positive integer, indicating that the energy generating system 102 may have any number of generators, such as one, two, three, or more. When four generators 103 are used, each having a maximum power output of 100 kW, the cumulative power output of the energy generating system is 400 kW, for example. In some embodiments the energy generating system 102 includes one generator 103 for each zone 111 of the multi-zone applicator 110 shown in FIG. 1. In other embodiments the quantity of generators can be a fraction or multiple of the number of zones 111 of the multi-zone applicator 110.

When the generators 103 are operating, the generators 103 generate and output electromagnetic energy E into the energy transfer assembly 106. In this example the energy transfer assembly 106 includes a plurality of waveguides that direct the electromagnetic energy from the generators to the multi-zone applicator 110 (FIG. 1). An example of a waveguide is a hollow conductive metal or dielectric pipe. The waveguide contains the electromagnetic energy therein and guides the energy to propagate along a length of the waveguide and through an open end into the multi-zone applicator 110. In FIG. 1 the electromagnetic energy generated by each generator is represented by $E_1$, $E_2$, $E_3$, and $E_N$, where the subscript represents the generator (1, 2, 3, or N) that generated the energy.

In some embodiments the waveguides described herein including portions of the energy transfer system as well as portions of the multi-zone applicator 110 are made of metal. Examples of suitable metals include aluminum and stainless steel. The waveguides have internal dimensions having a width and a height. In some embodiments the width is in a range from about 4 inches to about 16 inches, or about 6 inches to about 12 inches. In some embodiments the width is about 9.75 inches. In some embodiments the waveguides have a height in a range from about 2 inches to about 12 inches, or about 3 inches to about 7 inches. In some embodiments the height is about 4.875 inches.

In one possible embodiment, the energy transfer assembly 106 includes a plurality of waveguide segments. Flanges are provided at ends of each waveguide segment for fastening waveguide segments together, such as using bolts and nuts placed through apertures in the flanges. In some embodiments the energy transfer assembly 106 is elevated, such as to allow the waveguides to be positioned along a ceiling of the processing facility 90 (FIG. 1). In another possible embodiment the energy transfer assembly 106 is arranged within a floor of the facility 90. The energy transfer assembly 106 can alternatively be positioned in any desired configuration within the facility.

As discussed above, the energy generating system 102 can be arranged a distance away from the multi-zone applicator 110, such as across a room, in different rooms, or in different buildings. FIG. 2 graphically illustrates the space between the energy generating system 102 and the multi-zone applicator 110 by a distance L. The distance L can be any desired distance in a range from 0 to 0.5 miles away or more. In some embodiments the distance L is greater than 10, 20, 50, 100, 200, 500, or 1,000 feet away.

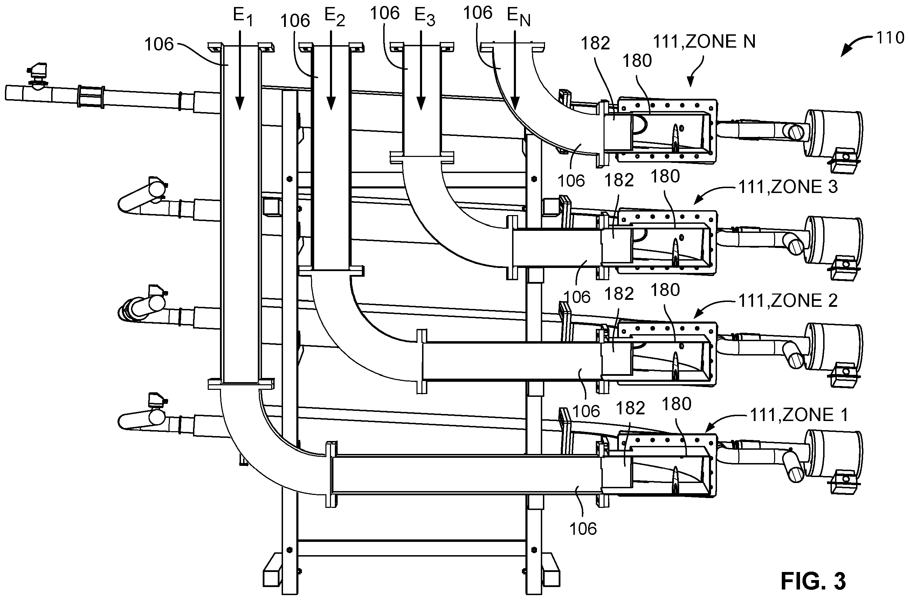

FIG. 3 illustrates another portion of the energy transfer assembly 106 where it connects to an example of the multi-zone applicator 110. FIG. 3 is a center cross-sectional view of the energy transfer assembly 106 and the example multi-zone applicator 110.

In this example, the energy transfer assembly 106 includes additional waveguide segments that continue to deliver energy E from the energy generating system 102 shown in FIG. 2 to the multi-zone applicator 110. Energy $E_1$ is delivered to application zone 1. Energy $E_2$ is delivered to application zone 2. Energy $E_3$ is delivered to application zone 3. Energy $E_N$ is delivered to application zone N.

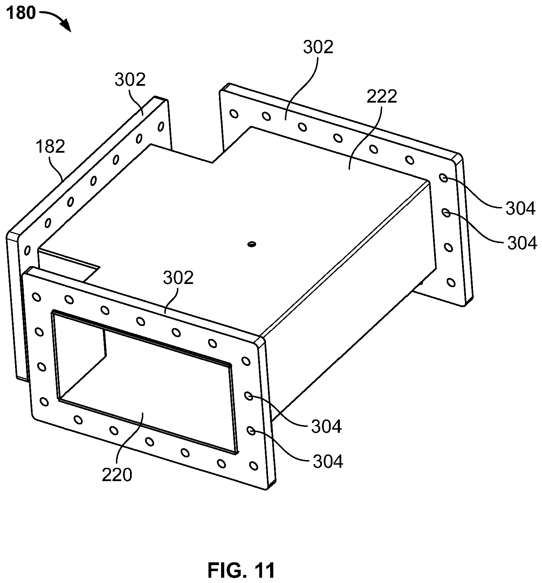

In some embodiments the energy transfer assembly 106 is connected to input ports of the multi-zone applicator 110. In some embodiments each application zone 111 includes an electromagnetic splitter 180. In the illustrated example, the energy transfer assembly terminates at and is connected to an input port 182 of the splitter 180. An example of the splitter 180 is illustrated and described in further detail herein with reference to FIG. 11.

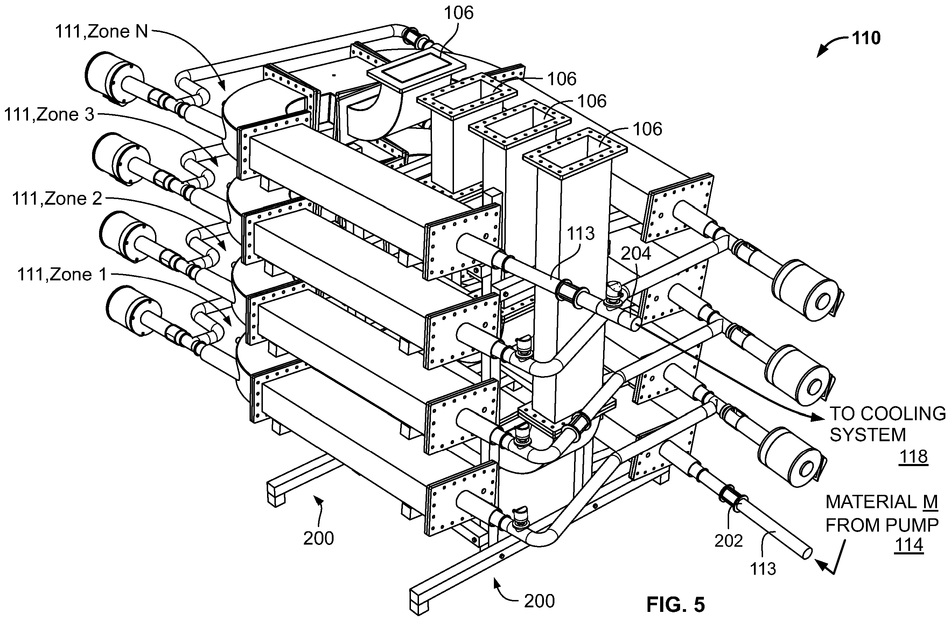

FIGS. 4 and 5 illustrate two isometric side views of an example multi-zone applicator 110 and portions of the energy transfer assembly 106. In this example the multi-zone applicator 110 includes a stand 200, a material input 201, a material output 203, the material conduit 113, and application zones 111 (including zones 1, 2, 3, ... N).

Material M from the pump 114 is delivered to the multi-zone applicator 110 into the material input 201. The material M flows through the material conduit 113 which passed through each of the application zones 111. It then exits the multi-zone applicator 110 at the material output 203 and proceeds to further components of the processing line 104, such as to the cooling system 118.

At least some of the material conduit 113 is made of a material that is transparent to electromagnetic energy, so that as the material passes through the application zones 111 the material is exposed to the electromagnetic energy. Several examples of materials that are transparent to electromagnetic energy include ceramics (such as Alumina ceramic), glass (such as Borosilicate glass), and plastic polymers (such as Teflon, polypropylene, polysulfone, polyetheretherketone (PEEK), and polyetherimide (Ultem)). The interaction between the material M and the electromagnetic energy typically causes heating of the material M.

In some embodiments the material conduit 113 is arranged and positioned within the multi-zone applicator 110 such that it has a continuously upward slope, such that absent pressure from the pump 114, the material M would be pulled by gravity backwards away from the material output 203. This prevents material M from reaching the material output 203 without being fully processed through all of the application zones 111, such as during a temporary loss of power, for example. In contrast, a downward slope could potentially allow the material to flow forward toward the material output 203 even when the pump 114 and/or the multi-zone applicator were not operating, under the force of gravity.

In some embodiments the material conduit 113 the continuously upward slope of the material conduit 113 through the multi-zone applicator 110 is in a range from about 1 degree to about 20 degrees, or in a range from about 1 degree to about 10 degrees, or in a range from about 1 degree to about 6 degrees. In some embodiments the continuously upward slope of the material conduit 113 is in a range from 1 degree to 6 degrees. In some embodiments the slope of the material conduit is not greater than 20 degrees, or is not greater than 10 degrees, or is not greater than 6 degrees. In some embodiments the continuously upward slope continues from the material input 201 to the material output 203 of the multi-zone applicator 110. In some embodiments the continuously upward slope is constant for the multi-zone applicator 110.

Although other shapes are also possible, the example multi-zone applicator 110 has a material conduit 113 that extends in a rectangular helical shape, starting at the lowest point at the material input 201 and advancing in a generally rectangular path defined by each zone 111 of the multi-zone applicator, while maintaining the continuously upward slope until it exits at the material output 203.

Embodiments of the multi-zone applicator 110 can be designed to have any number of zones including one, two, three, four, five, six, or more. Additionally, even when the multi-zone applicator 110 has a certain number of zones, such as four, each zone does not need to be activated if the particular material being processed does not require as many zones. For example, zones 1-3 can be used while the generator for the fourth zone can be turned off, such that the fourth zone is not activated. In such a case, the material conduit 113 can still route the material through the fourth zone if desired, or alternatively the material conduit 113 can be rerouted to bypass the fourth zone and advance directly to subsequent components such as the cooling system 118. In this way the multi-zone applicator is very flexible and can be quickly adjusted or customized for processing of a different material when needed, without requiring any or substantial changes be made to the multi-zone applicator 110.

Additionally, a benefit of some example embodiments is that the multi-zone applicator 110 can have as many zones 111 added as desired, without increasing the footprint of the multi-zone applicator. Referring to FIG. 4, the width W1 and length L1 remain fixed regardless of how many zones 111 are stacked on top, and only the height changes. Therefore the number of zones may be limited only by the height of the processing facility 90.

Additionally, then, it can be appreciated that another benefit of at least some embodiments is the compact design of each zone 111 in all dimensions, but particularly in its height. While some applicators can have other shapes, such as an upright S-shaped configuration that does not have a compact height, the configuration illustrated in FIGS. 4-5 (and elsewhere herein) includes zones 111 having generally a horizontal U-shaped configuration (not including the material conduit 113), which is slightly skewed in some embodiments in order to maintain the desired continually upward slope of the material conduit 113. Therefore, in this example the height of each

zone ($H_1$, $H_2$, $H_3$, ... $H_N$) is much less than the width or the length, allowing for a fixed footprint and allowing the option for many zones 111 to be stacked on top of each other within the typical processing facility 90.

The total height H of the multi-zone applicator 110 is equal to (or approximately equal to) the sum of the height of each of the zones (H = $H_1$ + $H_2$ + $H_3$ . + $H_N$).

Also, the height of each zone is typically equal, and therefore the total height can also be calculated as the product of a height (e.g., $H_1$) of any of the zones 111 and the number of zones N. In some embodiments the height (e.g., $H_1$) of each zone 111 is less than, or much less than, the width W1 and the length L1.

In some embodiments the height (e.g., $H_1$) of each zone 111 is less than about 30% of the length of the zone L1, or in a range from about 15 to about 25%. In some embodiments the height (e.g., $H_1$) of each zone 111 is less than about 20% of a width W1 of the zone 111, or in a range from about 10% to about 20%. In at least one example, the height $H_1$ (including four application zones 111) is about 81 inches (about 2 meters), the length L1 is about 157" (about 4 meters) and the width W1 is about 75 inches (about 1.9 meters). Other embodiments can have other sizes. As discussed herein, the height can be taller or shorter depending on the number of zones 111 utilized in the particular implementation.

However, the multi-zone applicator 110 can be scaled up or down in other embodiments depending on various factors such as the type and characteristics of the material to be processed, the volume of the material, the amount of energy that needs to be applied to the material in order to complete the desired processing of the material, and whether the multi-zone applicator 110 is to be used for large-scale commercial production, small-scale production, laboratory or experimental testing, or demonstration purposes, for example. Scaling of the power output of the energy generating system 102 (FIG. 2) can similarly be performed.

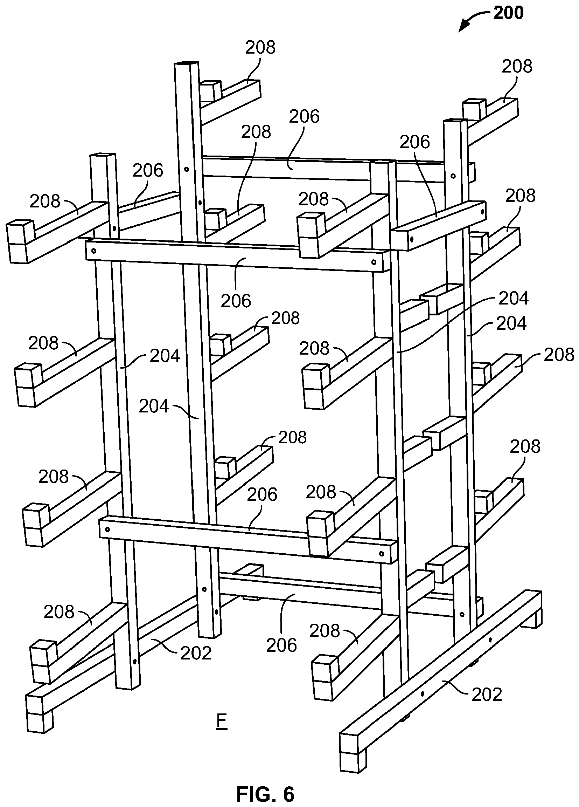

FIG. 6 is a perspective view of an example stand 200 of a multi-zone applicator 110. In this example the stand 200 includes a base 202, vertical supports 204, cross support members 206, and applicator supports 208.

The example stand 200 has a frame structure that is configured to be placed on a floor F and to support the other components of the multi-zone applicator 110 in a spaced relationship above the floor F.

The frame structure is formed of square metal tubing or beams, such as having a cross-sectional dimension in a range from about 1-3 inches, or about 2 inches.

The base 202 includes feet that rest on the floor F.

The vertical supports 204 extend vertically upward from the base.

The cross support members 206 extend between the vertical supports 204 to form a rigid frame structure that resists twisting and bending.

The applicator supports 208 are connected to the vertical supports 204 and extend outward from the vertical supports 204. The applicator supports 208 include flat top surfaces that are arranged and positioned so that the applicators for each application zone 111 (e.g., FIGS. 4-5) can be placed on and rest upon the top surfaces. Stops are positioned on the applicator supports 208 and extend vertically upward from the top surfaces which are positioned to prevent the applicators from sliding off of the top surfaces. The applicators can be placed on the top surfaces where they are held by the force of gravity. Additionally, flanges on edges of the applicators also help to prevent the applicators from sliding off of the top surfaces. In embodiments in which the applicators are supported by resting on top of the top surfaces of the applicator supports, the multi-zone applicator can be easily cleaned, or the configuration of the multi-zone applicator can be easily modified because of the absence of fasteners holding the applicators on the applicator supports—the applicators can simply be lifted off.

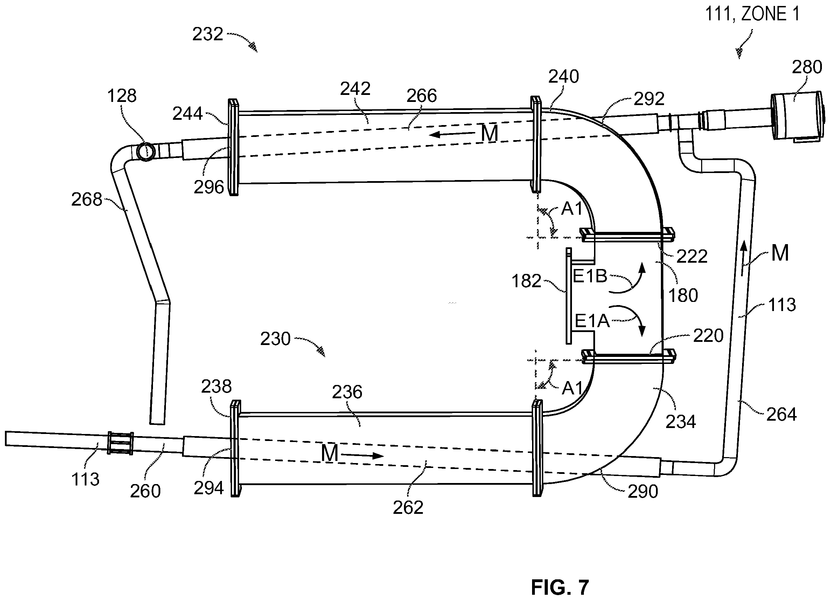

FIGS. 7-10 illustrate an example of the application zone 111 of the multi-zone applicator 110 shown in FIGS. 4-5.

FIG. 7 is a top view of the example application zone 111, and more specifically an example of the zone 1 of the multi-zone applicator 110 shown in FIGS 4-5. In this example, the application zone 111 includes the splitter 180, an applicator 230, an applicator 232, and the material conduit 113. The example splitter 180 includes the input port 182 and output ports 220 and 222. The example applicator 230 includes an elbow waveguide 234, a straight waveguide 236, and a reflector plate 238. The example applicator 232 includes an elbow waveguide 240, a straight waveguide 242, and a reflector plate 244. The material conduit 113 includes an input conduit 260, an applicator conduit 262, an intrazone transfer conduit 264, an applicator conduit 264, and a cross zone transfer conduit 268. Also shown in FIG. 7 are examples of the temperature sensor 128 and the mixer 280.

The splitter 180 is a waveguide that includes the input port 182 that is configured to be connected to a waveguide of the energy transfer assembly 106. The input port 182 receives the energy E1 generated by the generator 1 of the energy generating system 102 (shown in FIGS. 1-3). The splitter 180 then divides the energy into a plurality of portions and delivers the divided energy portions to the applicators. The illustrated example shows a splitter that divides the energy into two portions E1A and E1B. The splitter 180 includes the output port 220 that is connected to an end of, and delivers energy portion E1A into, applicator 230. The splitter 180 also includes the output port 222 that is connected to an end of, and delivers energy portion E1B into, applicator 232. An example of the splitter is illustrated and described in further detail with reference to FIG. 11.

One of the advantages of using the splitter 180 is that it allows a higher power generator 103 to be used to power the application zone 111. As discussed elsewhere herein, the inventors have found that higher powered applicators are often less expensive per unit power than lower powered applicators, and more particularly that a 100 kilowatt generator is currently thought by the inventors to be the most cost effective generator. However, for many processing operations a 100 kilowatt generator is too much power. Therefore, the splitter 180 can be used to reduce the power actually applied to the material by splitting it into two or more portions containing only a corresponding fraction of the power (e.g., one half, one third, one quarter, etc.). Thus, in the illustrated example when the energy E1 is 100 kilowatts, the energy portions delivered to the applicators 230 and 232 are 50 kilowatts.

It is also possible in some embodiments for the splitter 180 to be designed to divide the energy into two or more portions, such as two, three, four, or more. Further, some embodiments do not include a splitter, and instead the input port 182 is an input port of a single applicator, which receives the entire energy E1 directly from the energy transfer assembly 106.

In the same way that energy can be delivered directly to the applicator, or split into two or more portions, the zone 111 of the multi-zone applicator 110 can also include one or more applicators. In the illustrated example, the zone 111 includes two applicators 230 and 232. Other embodiments can have other quantities of applicators, such as one, three, four, or more.

The applicators 230 and 232 are formed of waveguides, and in this example include an elbow waveguide 234, 240, and a straight waveguide 236, 242, respectively. The elbow waveguides 234 and 240 are connected to the output ports 220 and 222 of the splitter 180, and curve at an angle A1 to redirect the energy portions by that angle. In this example the angle A1 is 90 degrees. Other angles can be used in other embodiments. An advantage of using an elbow waveguide 234, 240 is that it helps to reduce the overall footprint of the multi-zone applicator 110 by reducing the width W1 (shown in FIG. 4). Another advantage is that it reduces the length of material conduit 113 that may otherwise be required to transfer the material between application zones.

A further benefit of the elbow waveguides 234 and 240 is that it provides space along an outer side edge of the elbow waveguides 234 and 240 where a point of exit 290 or entry 292 can be selected from numerous possible arrangements. For example, the points of exit 290 and entry 292 can be selected to be adjacent the outer side wall of the applicators 230 and 232. The energy density is typically greatest in the centers of the waveguides and therefore the material can be more gradually introduced to or removed from the energy by arranging the points of exit and entry 292 away from the center of the waveguides. At the distal ends of the applicators 230 and 232 the same can be accomplished by arranging the points of entry 294 and exit 296 near to the opposite inner side wall of the waveguides 236 and 242, if desired. Alternatively, the energy E1A and E1B may be sufficiently attenuated by the time it reaches these distal ends of the applicators 230 and 232 that maximum exposure is desired, in which case the points of entry 294 and exit 296 can be arranged at the center of the waveguides 236 and 242, as in the example shown in FIGS. 7-10.

The elbow waveguides 234 and 240 deliver the energy portions E1A and E1B into the respective waveguides 236 and 242 which constitute the primary regions where the material M is exposed to the energy portions E1A and E1B.

In some embodiments the various waveguides can be connected together using a fastener, such as bolts and nuts arranged through apertures in the end flanges of each waveguide. This also allows for easy adjustment, modification, customization, removal, or cleaning of the waveguides by allowing them to be easily disconnected at any one or more points by simple disconnection of the fasteners securing them together.

In some embodiments any energy remaining at the distal ends of the applicators 230 and 232 could be directed into a water load where it is dissipated to prevent reflection of the energy into the applicator, in which case all remaining energy would be waste energy resulting in an undesirable inefficiency in the multi-zone applicator 110.

In another embodiment, such as shown in FIG. 7, a reflector plate 238, 244 is arranged at the distal ends of the applicators 230 and 232 to reflect any remaining energy back into the applicators 230 and 232 where it is again used to expose the material M.

The reflector plates 238, 244 have been found to result in greatly improved efficiencies. For example, an applicator with a water load termination may have 60-70 percent efficiency, whereas the applicator 230, 232 with a reflector plate 238, 244 may have 90 percent or more, or 95% or more efficiency. In some embodiments the applicator 230, 232 has about 97% efficiency. This is in sharp contrast to other heating techniques. For example, boilers used for canning can have efficiencies from about 10% to about 35%.

The material is supplied to and guided through the application zone 111 by the material conduit 113. In this example the material conduit 113 includes multiple segments including the input conduit 260, the applicator conduit 262, the intrazone transfer conduit 264, the applicator conduit 264, and the cross zone transfer conduit 268.

The material arrives at the application zone 111 through the input conduit 260. The input conduit 260 is connected to the applicator conduit 262.

The applicator conduit 262 is made of a material that is transparent to the electromagnetic energy. The applicator conduit 262 enters the applicator 230 through the point of entry 294 through the reflector plate 238, and then extends through the applicator waveguide 236 and into the elbow waveguide 234. The material M passing through the applicator conduit 262 is exposed to the portion E1A of the electromagnetic energy in the applicator 230. The applicator conduit 262 then exits the elbow waveguide 234 at the point of exit 290. The distal end of the applicator conduit is connected to the intrazone transfer conduit 264.

The intrazone transfer conduit 264 connects the applicators 230 and 232, which are both part of the same application zone 111. The intrazone transfer conduit 264 is connected to and conveys the material from the applicator conduit 262 to the applicator conduit 266 where it enters the applicator 232. In some embodiments the mixer 280 is arranged at the joint between the conduits 264 and 266, and operates to mix or agitate the material M as it enters the applicator conduit 266.

The applicator conduit 266 enters the applicator 232 through the point of entry 292 of the elbow waveguide 240 and then passes through the applicator waveguide 242 where, similar to the applicator conduit 262, the material M is exposed to the portion E1B of the electromagnetic energy. The applicator conduit 266 is made of a material that is transparent to the electromagnetic energy so the exposure can occur. The applicator conduit 262 exits the applicator 232 and the application zone 111 through the point of exit 296 through the reflector plate 244. The distal end of the applicator conduit 262 is connected to the cross zone transfer conduit 268.

After the material M exits the applicator 232, in some embodiments a temperature sensor 128 is arranged on the cross zone transfer conduit to measure a temperature of the material M. The temperature reading is communicated from the temperature sensor 128 to the control system 108.

The cross zone transfer conduit 268 provided to connect adjacent zones of the multi-zone applicator 110, to convey the material M from one zone to another zone for further processing. In this example, the cross zone transfer conduit 268 of zone 1 acts as the input conduit 260 for the application zone 2, and the material M is delivered from the zone 1 to the zone 2 by the cross zone transfer conduit 268.

The intrazone transfer conduits 264 and cross zone transfer conduits can also be used to make a variety of modifications or customizations to a particular implementation of the multi-zone applicator 110. For example, additional components can be connected to the application zone using these conduits, as desired. Some examples of additional components include a mixer; a de-aerator; an injection port, such as for the addition of ingredients (flavors, probiotics, vitamins), fluids, chemicals, reactants, and the like; sample ports for removing samples of the material; equilibration hold tubes; a cooling system; and other desired components. The conduits can also be used as bypass conduits to bypass a particular zone if desired.

In some embodiments the portions of the material conduit 113 that pass through the applicators, such as including the applicator conduits 262 and 264, are formed of a material that is transparent to the electromagnetic energy generated by the energy generating system 102. In some embodiments the portions of the material conduit that do not pass through the applicators (including the input conduit 260 and a final output conduit, as well as the transfer conduits 264 and 268 and conduits between other components of the material processing system 100 (e.g., tanks, pumps, cooling systems, etc.) need not be made of such a material, and can instead be made of another material such as stainless steel tubing. In some embodiments the material conduit 113 has an internal diameter in a range from about 1 inch to about 3 inches, such as 1.5 inches or 2 inches.

In some embodiments each of the application zones 111 has the same or similar structure, and therefore this discussion of the example application zone 111 for zone 1 will not be repeated herein for each of the other zones 2, 3, ... N, understanding that the structure of such zones is the same as or similar to that discussed herein for zone 1.

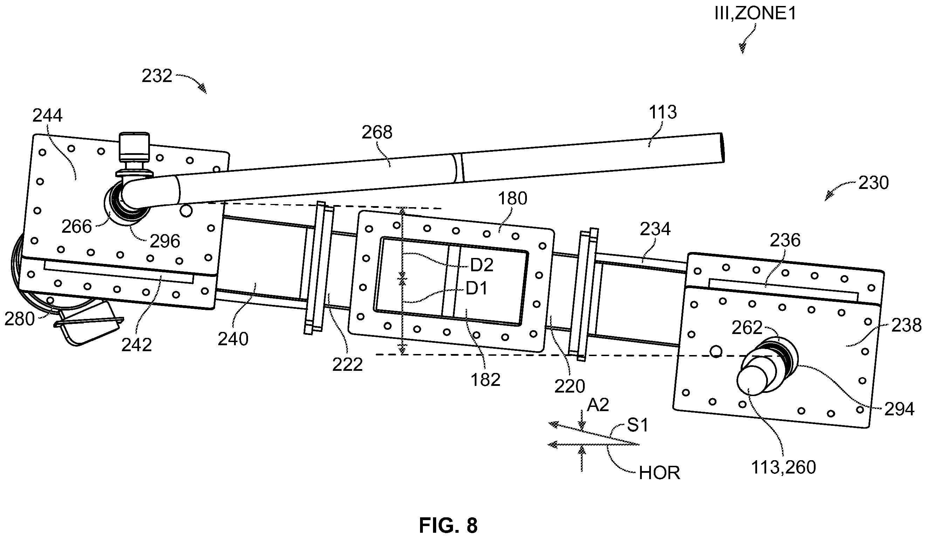

FIG. 8 is a side view of the example application zone 111 (e.g., zone 1) shown in FIG. 7.

The example application zone 111 has a shape, such as a slightly skewed horizontal U-shape. To illustrate an example of the skew, FIG. 8 illustrates a horizontal line HOR and a slope line S1 at an angle A2 to the horizontal line HOR. The slope line S1 corresponds to a slope of a plane passing through centers of the point of entry 294, input port 182, and point of exit 296. In some embodiments the slope line S1 has an angle in a range from about 5 degrees to about 25 degrees, and in some embodiments an angle in a range from about 10 degrees to about 20 degrees. In some embodiments the angle A2 is about 15 degrees. The skew can also be represented by a height differential. A distance D1 is a vertical height difference between a center of the point of entry 294 of applicator 230 and a center of the input port 182, and the distance D2 is a vertical height difference between a center of the input port 182 and the center of the point of exit 296. In some embodiments the distances D1 and D2 are in a range from about 2 inches to about 8 inches, or in a range from about 3 inches to about 6 inches. A total height difference from the point of entry 294 to the point of exit 296 of the zone 111 is the sum of D1 and D2 (in a range from about 4 inches to about 16 inches, or in a range from about 6 inches to about 12 inches), or twice either of the D1 or D2 measurements when the distances are equal.

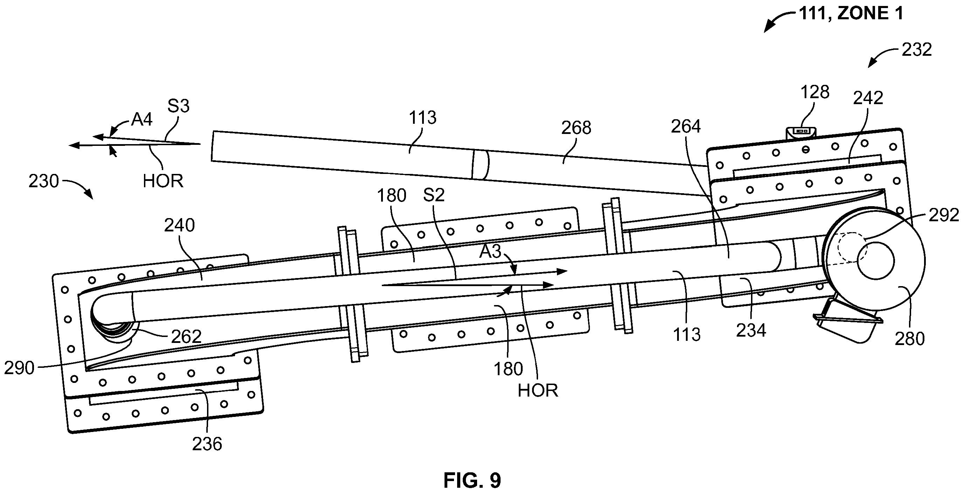

FIG. 9 is another side view of the example application zone 111 (e.g., zone 1) shown in FIG. 7.

The intrazone transfer conduit 264 extends from the point of exit 290 of the applicator 230 to the point of entry 292 of the applicator 232. The intrazone transfer conduit 264 has an upward slope S2 from a horizontal line HOR in a range from about 1 degree to about 20 degrees, or from about 1 degree to about 10 degrees. In some embodiments the intrazone transfer conduit 264 has a slope of about 4 degrees.

The cross zone transfer conduit 264 extends from the point of exit 296 (not visible in FIG. 9) of the applicator 232 to an point of entry 294 of a subsequent zone (e.g., zone 2). The cross zone transfer conduit 264 has an upward slope S3 from a horizontal line HOR in a range from about 1 degree to about 20 degrees, or from about 1 degree to about 10 degrees. In some embodiments the cross zone transfer conduit 264 has a slope of about 4 degrees.

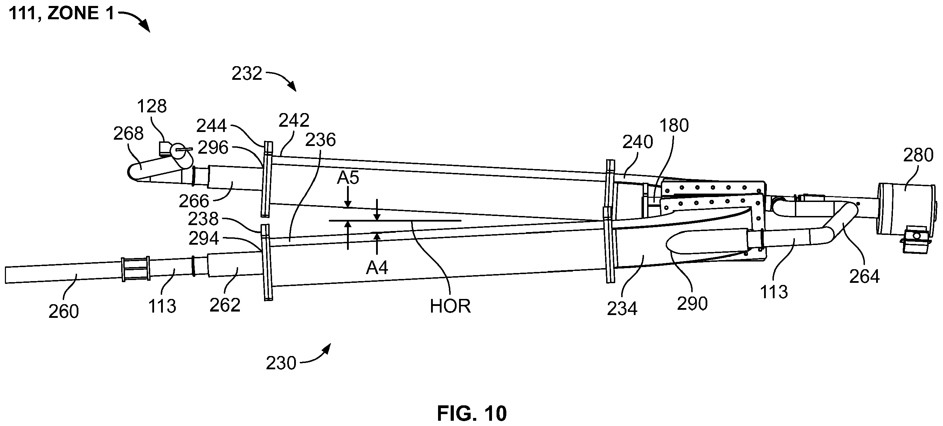

FIG. 10 is another side view of the example application zone 111 (e.g., zone 1) shown in FIG. 7.

In some embodiments the application zone 111 has a generally horizontal U-shaped configuration, which is slightly skewed. More specifically, the applicators 230 and 232 are skewed up and down from the horizontal, such that they both have an upward slope in the direction of the material flow through the material conduit 113. The horizontal line HOR is shown for reference. The slope of the applicator 230 is illustrated as an angle A4. The slope of the applicator 230 is also illustrated as an angle A5. In some embodiments the angles A4 and A5 are in a range from about 1 degree to about 20 degrees, or from about 1 degree to about 10 degrees. In some embodiments the applicators 230 and 232 have a slope angle A4 and A5 that matches a slope of the material conduit 113, as discussed herein. In some embodiments the slope angles A4 and A5 are in a range from about 1 degree to about 20 degrees, or in a range from about 1 degree to about 10 degrees, or in a range from about 1 degree to about 6 degrees. In some embodiments the slope is in a range from 1 degree to 6 degrees. In some embodiments the slope of the applicators is not greater than 20 degrees, or is not greater than 10 degrees, or is not greater than 6 degrees.

In some embodiments the material conduit 113 extends longitudinally through the applicators 230 and 232 in a direction parallel with the height of the applicators. Therefore, in some embodiments the slopes of the material conduit 113 (e.g., applicator conduits 262 and 266) are the same as angles A4 and A5.

In some embodiments at least some of the zones 111, applicators, and material conduits 113 extending through the zones 111 and applicators are said to be horizontal. As used herein, the term generally horizontal refers to an object which has a slope with respect to the earth or a supporting floor that is less than 45 degrees. On the other hand, a generally vertical object has a slope of greater than 45 degrees. A substantially horizontal object has a slope of less than 20 degrees. In other embodiments a substantially horizontal object has a slope of less than 10, or less than 6 degrees. Slopes of objects can be measured with respect to the earth or a level floor. The slopes described herein refer to the objects when assembled in their operational configuration, such as with the feet of the stand or other support structure resting on a level floor.

Although most of the examples illustrated herein involve horizontally arranged application zones, applicators, and material conduits, many of the principles described herein are also applicable to vertical, generally vertical, or substantially vertical zones, applicators, and material conduits, or such structures having combinations of horizontal and vertical components. As just one example, the capability to utilize multiple individually adjustable generators for separately controlling one or more zones or applicators is equally applicable to horizontal, vertical, or combinations of horizontal and vertical zones, applicators, and material conduits.

FIG. 11 is a perspective view of an example splitter 180. In this example the splitter includes an input port 182 and two output ports 220 and 222.

In some embodiments edges of the input and output ports 182, 220, and 222 include connection flanges 302, with apertures 304 extending therethrough. The flanges 302 are used for connecting the ports 182, 220, and 222 with other waveguides having similarly arranged flanges and ports, such as using a fastener. One example of a suitable fastener is a bolt and nut. Other fasteners can also be used in other embodiments. In some embodiments the flanges and/or locations of the apertures 304 are skewed to allow waveguides to be connected at the appropriate angles, such as discussed herein.

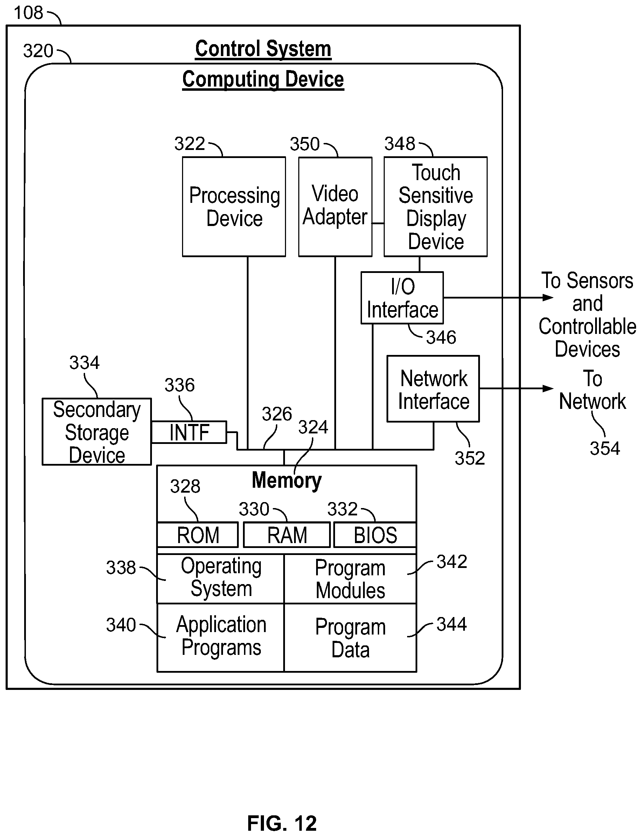

FIG. 12 is a schematic block diagram illustrating an example of the control system 108, shown in FIG. 1. In this example, the control system 108 includes a computing device 320. The computing device illustrated in FIG. 12 can be used to execute the operating system, application programs, methods, and software modules described herein.

The computing device 320 includes, in some embodiments, at least one processing device 322, such as a central processing unit (CPU). A variety of processing devices are available from a variety of manufacturers, for example, Intel or Advanced Micro Devices. In this example, the computing device 320 also includes a system memory 324, and a system bus 326 that couples various system components including the system memory 324 to the processing device 322. The system bus 326 is one of any number of types of bus structures including a memory bus, or memory controller; a peripheral bus; and a local bus using any of a variety of bus architectures.

Examples of computing devices suitable for the computing device 320 include a server computer, a desktop computer, a laptop computer, a tablet computer, a mobile computing device (such as a smart phone, an iPod® or iPad® mobile digital device, or other mobile devices), or other devices configured to process digital instructions.

The system memory 324 includes read only memory 328 and random access memory 330. A basic input/output system 332 containing the basic routines that act to transfer information within computing device 320, such as during start up, is typically stored in the read only memory 328.

The computing device 320 also includes a secondary storage device 334 in some embodiments, such as a hard disk drive, for storing digital data. The secondary storage device 334 is connected to the system bus 326 by a secondary storage interface 336. The secondary storage devices 334 and their associated computer readable media provide nonvolatile storage of computer readable instructions (including application programs and program modules), data structures, and other data for the computing device 320.

Although the exemplary environment described herein employs a hard disk drive as a secondary storage device, other types of computer readable storage media are used in other embodiments. Examples of these other types of computer readable storage media include magnetic cassettes, flash memory cards, digital video disks, Bernoulli cartridges, compact disc read only memories, digital versatile disk read only memories, random access memories, or read only memories. Some embodiments include non-transitory media. Additionally, such computer readable storage media can include local storage or cloud-based storage.

A number of program modules can be stored in secondary storage device 334 or memory 324, including an operating system 338, one or more application programs 340, other program modules 342 (such as the software engines described herein), and program data 344. The computing device 320 can utilize any suitable operating system, such as Microsoft Windows™, Google Chrome™, Google Android, Apple OS, Apple iOS, Linux, and any other operating system suitable for a computing device.

In some embodiments, a user provides inputs to the computing device 320 through one or more input devices, such as the touch sensitive display 348. Other input devices can also be used, such as a keyboard, mouse, pointer control device (such as a touch pad, touch stick, joy stick, etc.), microphone, and any other suitable input device. The input devices are often connected to the processing device 322 through an input/output interface 346 that is coupled to the system bus 326. Wireless communication between input devices and the interface 346 is possible as well, and includes infrared, BLUETOOTH® wireless technology, IEEE 802.11x Wi-Fi technology, cellular, or other radio frequency communication systems.

One or more input/output interfaces 346 can be used for communicating with external sensors and controllable devices of the material processing system. The input/output interface can include AC, DC, or digital input output interfaces, including for example USB and other i/o interfaces, and can also or alternatively include one or more communication devices such as a wireless communication device, wired network communication device (e.g., a modem or Ethernet communication device), or other wired communication devices (e.g., serial bus). Examples of sensors and other controllable devices are described herein, and include the energy generating system 102 and generators 103, the pump 114, the flow rate sensor 116, the temperature sensors 128, and the back pressure device 122, as shown in FIG. 1, or other sensors or controllable devices.

In this example embodiment, a display device 348, such as a monitor, liquid crystal display device, projector, or touch sensitive display device, is also connected to the system bus 326 via an interface, such as a video adapter 350. In addition to the display device 348, the computing device 320 can include various other peripheral devices (not shown), such as speakers or a printer.

When used in a local area networking environment or a wide area networking environment (such as the Internet), the computing device 320 is typically connected to a network 354 through a network interface 352, such as an Ethernet interface, or by a wireless communication device, such as using cellular or Wi-Fi communication.

The computing device 320 typically includes at least some form of computer readable media. Computer readable media includes any available media that can be accessed by the computing device 320. By way of example, computer readable media include computer readable storage media and computer readable communication media.

Computer readable storage media includes volatile and nonvolatile, removable and non-removable media implemented in any device configured to store information such as computer readable instructions, data structures, program modules or other data. Computer readable storage media includes, but is not limited to, random access memory, read only memory, electrically erasable programmable read only memory, flash memory or other memory technology, compact disc read only memory, digital versatile disks or other optical storage, magnetic cassettes, magnetic tape, magnetic disk storage or other magnetic storage devices, or any other medium that can be used to store the desired information and that can be accessed by the computing device 320. Computer readable storage media does not include computer readable communication media.

Computer readable communication media typically embodies computer readable instructions, data structures, program modules or other data in a modulated data signal such as a carrier wave or other transport mechanism and includes any information delivery media. The term "modulated data signal" refers to a signal that has one or more of its characteristics set or changed in such a manner as to encode information in the signal. By way of example, computer readable communication media includes wired media such as a wired network or direct-wired connection, and wireless media such as acoustic, radio frequency, infrared, and other wireless media. Combinations of any of the above are also included within the scope of computer readable media.

The computing device illustrated in FIG. 12 is also an example of programmable electronics, which may include one or more such computing devices, and when multiple computing devices are included, such computing devices can be coupled together with a suitable data communication network so as to collectively perform the various functions, methods, or operations disclosed herein.

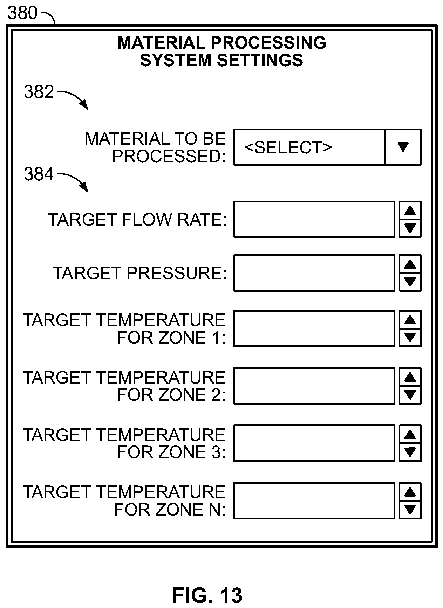

FIG. 13 is a screen shot of an example graphical user interface 380 of the control system 108, shown in FIGS. 1 and 12.

In some embodiments the graphical user interface 380 is generated on a display device by a computing device, such as the display device 348 and the computing device 320, shown in FIG. 12. In some embodiments data instructions are stored in at least one computer readable data storage device, which when executed by at least one computing device, cause the computing device to generate the graphical user interface 380. The graphical user interface 380 can be displayed with multiple windows or pages, in some embodiments, but in FIG. 13 is illustrated as being displayed on a single window or page.

In this example, the graphical user interface 380 includes a material identification section 382 and a target definition section 384.

The material identification section 382 prompts the user to identify the type of material to be processed by the material processing system. In some embodiments a menu of options is provided for selection, and the user can review the list of materials and select one of the materials from the list.

In some embodiments, the materials contained in the list are associated with one or more material characteristics. An example of a material characteristic is a heat capacity of the material. In another possible embodiment, the material is associated with a recipe, which includes predefined material processing system settings for processing the selected material. Upon selection of that material, the predefined material processing system settings are automatically filled into the graphical user interface 380 fields where the user can review the settings, accept the settings, or adjust the settings.

Alternatively, if the material is not available in the menu, some embodiments provide an option to add a new material. Upon selection of the add new material option, the user is prompted to provide a name for the material and to identify one or more material characteristics, in some embodiments.

The target definition section 384 prompts the user to identify a plurality of material processing targets to be used during the processing of the material. In this example the target definition section 384 includes prompts and data entry fields for target flow rate, target pressure, and target temperatures for each zone (e.g., zones 1-N).

To define a target value, the user selects the corresponding data entry field and enters the desired value, or utilizes the up and down arrows to increment an existing value up or down, for example.

In some embodiments (not shown in FIG. 13) target temperatures are provided for at least two stages of operation. A first stage of operation involves a sterilization stage, and the second stage of operation involves a material processing stage. During the sterilization stage, the material processing system 100 is operated to sterilize the system in preparation for material processing. Often higher temperatures may be used in order to ensure that the material conduit is clean and clear of any contaminants, and therefore a first set of sterilization temperatures can be defined for use during the sterilization stage. Then after the sterilization stage is completed, the system advances to the material processing stage in which the material processing target temperatures that are separately defined, are used for processing the material.

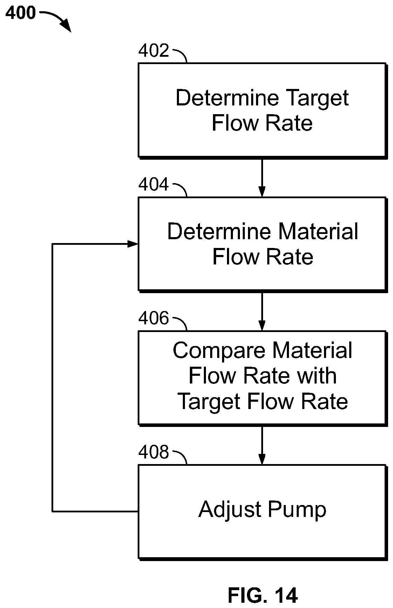

FIG. 14 is a flow chart illustrating an example method 400 of controlling a material flow rate. In some embodiments the method 400 is performed by the control system 108. In this example, the method 400 includes operations 402, 404, 406, and 408.

The operation 402 is performed to determine a target flow rate. In some embodiments the operation 402 involves receiving the target flow rate from the user through the graphical user interface 380, shown in FIG. 13. In another possible embodiment the target flow rate is retrieved from a database based on a material to be processed, such as a material selected through the graphical user interface 380, shown in FIG. 13.

The operation 404 is performed to determine a material flow rate. In one example the material flow rate is the rate of flow of the material through the material conduit 113 described herein, such as measured by the flow rate sensor 116, shown in FIG. 1. In some embodiments the material flow rate is a current flow rate.

The operation 406 is performed to compare the material flow rate with the target flow rate. The operation 406 determines for example, a deviation between the material flow rate and the target flow rate. In some embodiments the deviation is logged in a computer readable data storage device, such as along with a time stamp indicating a time at which the deviation was identified. In some embodiments an alert operation occurs when the deviation is greater than a threshold value. The alert can include an audible alert, a visible alert on the graphical user interface of the display device or by illuminating or activating a visible light, or sending a message (text, e-mail, etc.). Alerts can similarly be generated when a deviation greater than a threshold value is identified for any of the material processing targets described herein.

The operation 408 is performed to adjust a pump. For example, when the material flow rate and the target flow rate are not equal, or when the deviation is outside of a maximum deviation range, the operation of the pump is adjusted in an effort to make the material flow rate match the target flow rate. In some embodiments the operations 404, 406, 408 are repeated to continue monitoring and adjusting the flow rate.

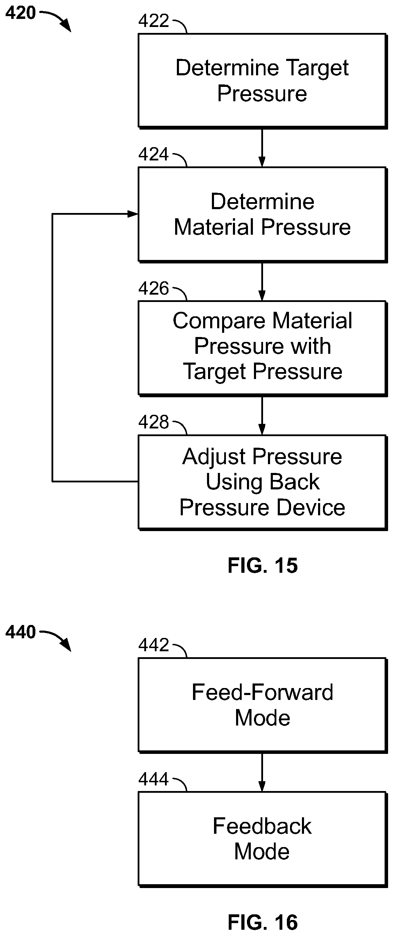

FIG. 15 is a flow chart illustrating an example method 420 of adjusting a pressure of the material processing system. In some embodiments the method 420 is performed by the control system 108, shown in FIGS. 1 and 12. In this example, the method 420 includes operations 422, 424, 426, and 428.

The operation 422 is performed to determine a target pressure. In some embodiments the target pressure is determined by receiving a target pressure from the user through the graphical user interface 380, shown in FIG. 13. In another possible embodiment the target pressure is retrieved from a database based on the material to be processed.

The operation 424 is performed to determine a material pressure. In one example the material pressure is a pressure within the material conduit 113 (FIG. 1), and therefore a pressure of the material being processed. In some embodiments the material pressure is determined by reading a value from the pressure sensor 120, shown in FIG. 1.

The operation 426 is performed to compare the material pressure with the target pressure. The operation 426 determines for example, a deviation between the material pressure and the target pressure. In some embodiments the deviation is logged, such as along with a time stamp indicating a time at which the deviation was identified. An alert can also be generated in some embodiments.

The operation 428 is performed to adjust the pressure. For example, when a deviation is identified, the operation of a pressure adjustment device, such as a back pressure device, is adjusted to change the pressure up or down based on the determined deviation in an effort to make the material pressure equal the target pressure within at least a predetermined range.

An advantage of applying a back pressure to the material is that it can allow higher temperatures to be obtained without the material undergoing a phase change to gas. A temperature, pressure, phase diagram (or associated data) can be used to determine a pressure needed for a given material in order to maintain the material in the liquid phase. For example, low acid material can require sterilization temperatures in excess of 121 degrees C., and typically reaching up to 140 degrees C. This is above the flash point of liquids and thus requires a significant amount of pressure to keep the material in a liquid state. Typical pressures for a low acid material are in a range from about 60 PSI to about 80 PSI. Further, in some embodiments the control system also monitors the pressure and compares the pressure to a maximum pressure to ensure that the pressure does not exceed the maximum pressure.

FIG. 16 is a flow chart illustrating a method 440 of operating a material processing system. In some embodiments the method 440 is performed by a control system 108, such as shown in FIG. 1. In this example the method includes operations 442 and 444.

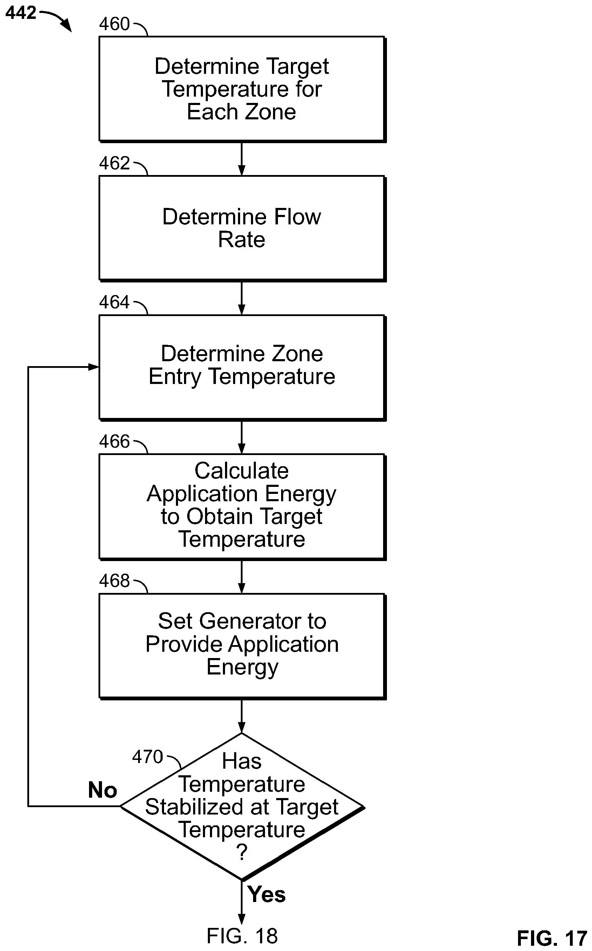

The operation 442 is performed to operate the material processing system in a feed-forward mode. The feed-forward mode utilizes a temperature sensor positioned upstream of an application zone (or prior to an applicator) to determine a temperature of the material as it enters the application zone or applicator, and uses that temperature to predict an amount of energy needed in order to raise the material temperature to a target temperature, and the generator is then controlled in order to output the predicted amount of energy. An example of the operation 442 is illustrated and described in more detail with reference to FIG. 17. In some embodiments the operation 442 continues until the material temperature after the zone or applicator has stabilized.

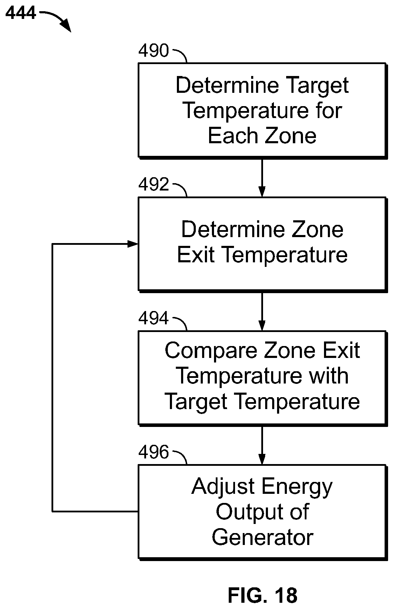

The operation 444 is performed to operate the material processing system in a feedback mode. The feedback mode utilizes a temperature sensor positioned downstream of an application zone or applicator to determine a temperature of the material after it exits the application zone or applicator. The measured material temperature is then used in a feedback loop to adjust the output of the generator based on a comparison of the material temperature and a target temperature. An example of the operation 444 is illustrated and described in further detail with reference to FIG. 18.

FIG. 17 is a flow chart illustrating an example method of controlling a material processing system. FIG. 17 is also an example of the feed-forward mode of operation 442, shown in FIG. 16. In this example the method/operation 442 includes operations 460, 462, 464, 466, 468, and 470.

The operation 460 is performed to determine a target temperature for each zone. In some embodiments determining the target temperature for each zone is performed by receiving the target temperatures from a user, such as through the graphical user interface 380 (e.g., target temperature for zone 1, 2, 3, N), shown in FIG. 13. In another possible embodiment, the target temperatures are retrieved from a database, such as based on a recipe defined for the material to be processed. In some embodiments the material to be processed is identified by the user through the graphical user interface 380, shown in FIG. 13.

The operation 462 is performed to determine a material flow rate. In some embodiments the flow rate is measured by the flow rate sensor 116, shown in FIG. 1.

The operation 464 is performed to determine a zone entry temperature. The zone entry temperature is a temperature of the material measured by a temperature sensor located upstream from the zone or applicator, and therefore corresponds with the temperature of the material as it enters the zone or applicator.

The operation 466 is performed to calculate an application energy required in order to obtain the target temperature for that zone or applicator. The operation 466 can utilize an energy balance equation in order to determine or estimate the appropriate amount of energy to be applied to the material. As one example, the energy can be computed using Equation 1:

EQUATION 1:

$$Q = m * Cp * dT$$

In Equation 1, Q is the energy, m is the mass flow rate, Cp is a heat capacity of the material, and dT (or delta T) is the change in temperature. The change in temperature (dT) is equivalent to the temperature of the material as it exits the zone or applicator minus the temperature of the material as it enters the zone or applicator. Further, the temperature of the material as it exits the zone or applicator is the target temperature (operation 460), whereas the temperature of the material as it enters the zone or applicator is the temperature determined in operation 464.

Once the application energy has been determined, the operation 468 is performed to set the generator to provide the application energy. In some embodiments only the generator that is coupled with the zone or applicator, to provide electromagnetic energy to the zone or applicator, is adjusted. In some embodiments the power output of the generator is selected. The power output value that is required to obtain the desired energy output from the generator can be computed directly, or determined from a lookup table, for example, based on the application energy determined in operation 466. In some embodiments the power output value is a percentage of the maximum power output.

If the method 440, shown in FIG. 16, is being performed, then an operation 470 can further be performed to determine when to switch between the feed-forward mode and the feedback mode. The operation 470 is performed to determine whether the temperature of the material has stabilized at the target temperature. In some embodiments the operation 470 involves receiving a temperature measurement from a temperature sensor downstream of the zone or applicator, and determining when the temperature has reached the target temperature, or when the temperature has stabilized, such as by remaining at the same temperature for a period of time, or a combination of both. If so, the material processing system is then transitioned to the feedback mode, such as shown in FIG. 18. Otherwise, the material processing system continues operating in the feed-forward mode and returns to operation 464.

In some embodiments the method/operation 442 is performed for a multi-zone applicator 110, with at least operations 464, 466, 468, and 470 being performed for each zone, and is also used to control the corresponding one or more generators for each zone.

FIG. 18 is a flow chart illustrating an example method of controlling a material processing system. FIG. 18 is also an example of the feedback mode of operation 444, shown in FIG. 16. In this example, the method/operation 444 includes operations 490, 492, 494, and 496.

The operation 490 is performed to determine a target temperature for each zone.

The operation 492 is performed to determine a zone exit temperature. In some embodiments the operation 492 involves receiving or reading a temperature downstream of the zone or applicator to determine a temperature of the material as it exits the zone or applicator.

The operation 494 is performed to compare the zone exit temperature with the target temperature. In some embodiments a differential is computed between the zone exit temperature and the target temperature.

The operation 496 is then performed to adjust the application energy / energy output of the generator associated with the zone or applicator. For example, when a deviation is identified, the output of the generator is adjusted to increase or decrease the energy output in order to increase or decrease the material temperature.

In some embodiments the method/operation 444 is performed for a multi-zone applicator 110, with at least operations 492, 494, and 496 being performed for each zone, and is also used to control the corresponding one or more generators for each zone.

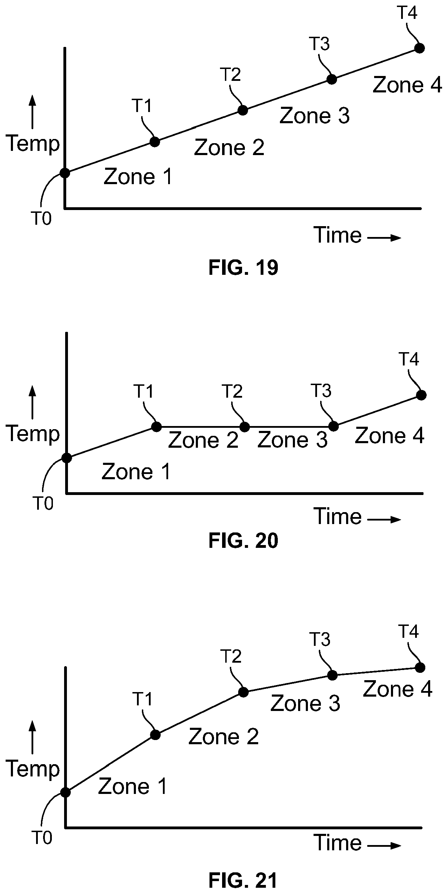

FIGS. 19-21 illustrate example material temperature profiles that can be achieved using the material processing system 100 as controlled by the control system 108 as described herein. These examples illustrate an embodiment in which the material processing system 100 includes a multi-zone applicator having four zones.

FIG. 19 illustrates a temperature profile having a linear ramp profile. In this example, the temperatures for the zones (T1, T2, T3, and T4) are selected so that the material temperature is raised at a steady rate from an initial temperature (T0) to a final temperature (T4).

FIG. 20 illustrates a temperature profile having a ramp, soak, ramp profile, including a soak period. In this example, the material is brought up to a first temperature (T1) and is then held at that temperature for a period of time (through zones 2 and 3), and finally is heated to the final temperature (T4). A temperature profile that holds a steady temperature is referred to as a soak period. One example of a material that benefits from a soak period as shown in FIG. 20 is a material containing starch. The soak period allows time for the starches to bloom, followed by a final heating to the final temperature in order to sterilize the product.

FIG. 21 illustrates a temperature profile having a tapered profile. The tapered profile initially provides rapid heating to T1, and then gradually reduces the rate of temperature increase. As the temperature increases, the control system reduces the rate of increase so that it slowly reaches the final highest temperature T4. An advantage of the tapered profile is that it reduces energy density applied to the material while the material is reaching its highest temperature, and reduces burning or fouling that may otherwise occur for sensitive materials.

In some embodiments the temperature profiles shown in FIGS. 19-21 (and many other variations) can be achieved by simply adjusting the target temperatures through the graphical user interface shown in FIG. 13, for example, without requiring any physical changes to be made to the structure or configuration of the material processing system 100 or multi-zone applicator 110.

The various embodiments described above are provided by way of illustration only and should not be construed to limit the claims attached hereto. Those skilled in the art will readily recognize various modifications and changes that may be made without following the example embodiments and applications illustrated and described herein, and without departing from the scope of the following claims.

**Claims**

1. A material processing system comprising:

    an energy generating system (102) comprising a plurality of electromagnetic energy generators (103);
    a pump (114) configured to provide a flow of material to be processed; and
    a multi-zone applicator (110) spaced from the energy generating system and comprising:

        a plurality of application zones (111), each of the application zones configured to receive electromagnetic energy generated by at least one of the plurality of generators (103); and
        a material conduit (113) coupled to the pump (114) to receive the flow of material and extending through the application zones (111), at least part of the material conduit (113) being transparent to the electromagnetic energy to allow the material to be exposed to the electromagnetic energy as the material passes through the application zones (111);

    wherein the application zones (111) have the same shape, are arranged in a common generally horizontal orientation, and are vertically aligned; the material processing system being **characterised in that** the material conduit (113) has a continuously upward slope throughout the multi-zone applicator.

2. The material processing system of claim 1, wherein the application zones (111) include a first application zone comprising:

    a first applicator;
    a second applicator; and
    a splitter (180) coupled between the first and second applicators and configured to divide and distribute the electromagnetic energy between the first and second applicators.

3. The material processing system of claim 2, wherein each of the applicators comprises an open end and a closed end, the open end configured to receive the electromagnetic energy distributed to it by the splitter, and the closed end comprising a reflector plate (244) configured to reflect the electromagnetic energy within the respective first and second applicators.

4. The material processing system of claim 3, wherein the reflector plate (244) comprises an aperture therein, and wherein the material conduit extends through the aperture.

5. The material processing system of claim 2, wherein the first and second applicators are waveguides.

6. The material processing system of claim 3, wherein the material conduit (113) is arranged to pass the material through the first applicator in a direction opposite to an electromagnetic energy application direction in the first applicator, and wherein the material conduit is arranged to pass the material through the second applicator in a same direction as the electromagnetic energy application direction in the second applicator.

7. The material processing system of claim 1, further comprising a plurality of temperature sensors (128), each of the temperature sensors configured to measure a temperature of the material after the material has passed through a respective application zone.

8. The material processing system of claim 1, wherein the continuously upward slope is in a range from 1 to 6 degrees.

**Patentansprüche**

1. Materialverarbeitungssystem, umfassend:

    Ein Energieerzeugungssystem (102), welches eine Vielzahl elektromagnetischer Energiegeneratoren (103) umfasst;
    Eine Pumpe (114), die ausgelegt ist, einen zu verarbeitenden Materialfluss bereitzustellen; und
    ein mehrzoniges Anwendungsgerät (110), das vom Energieerzeugungssystem beabstandet ist und umfasst:

        Eine Vielzahl von Anwendungszonen (111), wobei jede der Anwendungszonen ausgelegt ist, elektromagnetische Energie zu empfangen, die von zumindest einem der Vielzahl von Generatoren (103) erzeugt wurde; und

eine an die Pumpe (114) gekoppelte Materialleitung (113) zum Empfangen des Materialflusses und sich durch die Anwendungszonen (111) erstreckend, wobei zumindest ein Teil der Materialleitung (113) transparent für die elektromagnetische Energie ist, um dem Material zu ermöglichen, der elektromagnetischen Energie ausgesetzt zu werden, während das Material die Anwendungszonen (111) durchläuft;

wobei die Anwendungszonen (111) die gleiche Form aufweisen, in einer gemeinsamen generell horizontalen Orientierung angeordnet sind und vertikal ausgerichtet sind; wobei das Materialverarbeitungssystem **dadurch gekennzeichnet ist, dass** die Materialleitung (113) eine kontinuierlich ansteigende Steigung im ganzen mehrzonigen Anwendungsgerät aufweist.

**2.** Materialverarbeitungssystem nach Anspruch 1, wobei die Anwendungszonen (111) eine erste Anwendungszone einschließen, die umfasst:

Ein erstes Anwendungsgerät; ein zweites Anwendungsgerät; und einen Splitter (180), der zwischen den ersten und zweiten Anwendungsgeräten gekoppelt ist und ausgelegt ist, die elektromagnetische Energie zwischen den ersten und zweiten Anwendungsgeräten aufzuteilen und zu verteilen.

**3.** Materialverarbeitungssystem nach Anspruch 2, wobei jedes der Anwendungsgeräte ein offenes Ende und ein geschlossenes Ende umfasst, wobei das offene Ende ausgelegt ist, die elektromagnetische Energie zu empfangen, die vom Splitter an dieses verteilt wurde, und das geschlossene Ende eine Reflektorplatte (244) umfasst, die ausgelegt ist, die elektromagnetische Energie innerhalb der ersten und zweiten Anwendungsgeräte zu reflektieren.

**4.** Materialverarbeitungssystem nach Anspruch 3, wobei die Reflektorplatte (244) eine Öffnung drin umfasst, und wobei sich die Materialleitung durch die Öffnung erstreckt.

**5.** Materialverarbeitungssystem nach Anspruch 2, wobei die ersten und zweiten Anwendungsgeräte Wellenleiter sind.

**6.** Materialverarbeitungssystem nach Anspruch 3, wobei die Materialleitung (113) eingerichtet ist, das Material durch das erste Anwendungsgerät in eine Richtung entgegengesetzt zu einer Anwendungsrichtung elektromagnetischer Energie im ersten Anwendungsgerät zu leiten, und wobei die Materialleitung eingerichtet ist, das Material durch das zweite Anwendungsgerät in einer gleichen Richtung wie die Anwendungsrichtung elektromagnetischer Energie im zweiten Anwendungsgerät zu leiten.

**7.** Materialverarbeitungssystem nach Anspruch 1, das ferner eine Vielzahl von Temperatursensoren (128) umfasst, wobei jeder der Temperatursensoren ausgelegt ist, eine Temperatur des Materials zu messen, nach dem das Material eine jeweilige Anwendungszone durchlaufen hat.

**8.** Materialverarbeitungssystem nach Anspruch 1, wobei die kontinuierlich ansteigende Steigung in einem Bereich von 1 bis 6 Grad liegt.

**Revendications**

**1.** Système de traitement de matière comprenant :

un système de génération d'énergie (102) comprenant une pluralité de générateurs d'énergie électromagnétique (103) ; une pompe (114) configurée pour fournir un flux de matière à traiter ; et un applicateur multizone (110) espacé du système de génération d'énergie et comprenant :

une pluralité de zones d'application (111), chacune des zones d'application étant configurée pour recevoir de l'énergie électromagnétique générée par au moins l'un de la pluralité de générateurs (103) ; et un conduit de matière (113) couplé à la pompe (114) pour recevoir le flux de matière et s'étendant à travers les zones d'application (111), au moins une partie du conduit de matière (113) étant transparente à l'énergie électromagnétique pour permettre à la matière d'être exposée à l'énergie électromagnétique à mesure que la matière passe à travers les zones d'application (111) ;

dans lequel les zones d'application (111) ont la même forme, sont agencées dans une orientation commune généralement horizontale, et sont alignées verticalement ; le système de traitement de matière étant **caractérisé en ce que** le conduit de matière (113) a une pente continuellement ascendante tout au long de l'applicateur multizone.

**2.** Système de traitement de matière selon la revendication 1, dans lequel les zones d'application (111) comportent une première zone d'application comprenant :

un premier applicateur ;

un deuxième applicateur ; et

un séparateur (180) couplé entre les premier et deuxième applicateurs et configuré pour diviser et distribuer l'énergie électromagnétique entre les premier et deuxième applicateurs.

3.  Système de traitement de matière selon la revendication 2, dans lequel chacun des applicateurs comprend une extrémité ouverte et une extrémité fermée, l'extrémité ouverte étant configurée pour recevoir l'énergie électromagnétique qui lui est distribuée par le séparateur, et l'extrémité fermée comprenant une plaque de réflecteur (244) configurée pour réfléchir l'énergie électromagnétique au sein des premier et deuxième applicateurs respectifs.

4.  Système de traitement de matière selon la revendication 3, dans lequel la plaque de réflecteur (244) comprend une ouverture en son sein, et dans lequel le conduit de matière s'étend à travers l'ouverture.

5.  Système de traitement de matière selon la revendication 2, dans lequel les premier et deuxième applicateurs sont des guides d'onde.

6.  Système de traitement de matière selon la revendication 3, dans lequel le conduit de matière (113) est agencé pour faire passer la matière à travers le premier applicateur dans une direction opposée à une direction d'application d'énergie électromagnétique dans le premier applicateur, et dans lequel le conduit de matière est agencé pour faire passer la matière à travers le deuxième applicateur dans une même direction que la direction d'application d'énergie électromagnétique dans le deuxième applicateur.

7.  Système de traitement de matière selon la revendication 1, comprenant en outre une pluralité de capteurs de température (128), chacun des capteurs de température étant configuré pour mesurer une température de la matière après le passage de la matière à travers une zone d'application respective.

8.  Système de traitement de matière selon la revendication 1, dans lequel la pente continuellement ascendante est dans une plage de 1 à 6 degrés.

**FIG. 1**

**FIG. 2**

EP 3 412 115 B1

FIG. 3

FIG. 4

110

TO COOLING SYSTEM 118

MATERIAL M FROM PUMP 114

113

202

204

113

200

200

106

106

106

106

106

106

111, Zone N

111, Zone 3

111, Zone 2

111, Zone 1

FIG. 5

FIG. 6

**FIG. 7**

FIG. 8

FIG. 9

**FIG. 10**

**FIG. 11**

108

**Control System**

320

**Computing Device**

322
Processing Device

350
Video Adapter

348
Touch Sensitive Display Device

I/O Interface
346

To Sensors and Controllable Devices

334
Secondary Storage Device

336
INTF

326 324

Network Interface
352

To Network
354

**Memory**

328
ROM

330
RAM

332
BIOS

338
Operating System

342
Program Modules

340
Application Programs

344
Program Data

**FIG. 12**

380

MATERIAL PROCESSING
SYSTEM SETTINGS

382

MATERIAL TO BE
PROCESSED:   <SELECT> ▼

384

TARGET FLOW RATE:   ▲▼

TARGET PRESSURE:   ▲▼

TARGET TEMPERATURE
FOR ZONE 1:   ▲▼

TARGET TEMPERATURE
FOR ZONE 2:   ▲▼

TARGET TEMPERATURE
FOR ZONE 3:   ▲▼

TARGET TEMPERATURE
FOR ZONE N:   ▲▼

FIG. 13

**400**

402 — 
┌─────────────────────┐
│  Determine Target   │
│     Flow Rate       │
└─────────────────────┘

404 —
┌─────────────────────┐
│  Determine Material │
│     Flow Rate       │
└─────────────────────┘

406 —
┌─────────────────────┐
│  Compare Material   │
│   Flow Rate with    │
│  Target Flow Rate   │
└─────────────────────┘

408 —
┌─────────────────────┐
│    Adjust Pump      │
└─────────────────────┘

**FIG. 14**

420 ⤸

422 ⤷

```
┌─────────────────────┐
│  Determine Target   │
│     Pressure        │
└─────────────────────┘
```

424 ⤷

```
┌─────────────────────┐
│    Determine        │
│ Material Pressure   │
└─────────────────────┘
```

426 ⤷

```
┌─────────────────────┐
│  Compare Material   │
│   Pressure with     │
│  Target Pressure    │
└─────────────────────┘
```

428 ⤷

```
┌─────────────────────┐
│  Adjust Pressure    │
│   Using Back        │
│  Pressure Device    │
└─────────────────────┘
```

**FIG. 15**

440 ⤸

442 ⤷

```
┌─────────────────────┐
│   Feed-Forward      │
│      Mode           │
└─────────────────────┘
```

444 ⤷

```
┌─────────────────────┐
│    Feedback         │
│      Mode           │
└─────────────────────┘
```

**FIG. 16**

442

460

Determine Target
Temperature for
Each Zone

462

Determine Flow
Rate

464

Determine Zone
Entry Temperature

466

Calculate
Application Energy
to Obtain Target
Temperature

468

Set Generator to
Provide Application
Energy

470

Has
Temperature
Stabilized at Target
Temperature
?

**No**

**Yes**

FIG. 18

**FIG. 17**

444

490

Determine Target
Temperature for
Each Zone

492

Determine Zone
Exit Temperature

494

Compare Zone Exit
Temperature with
Target Temperature

496

Adjust Energy
Output of
Generator

FIG. 18

FIG. 19

FIG. 20

FIG. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20130122160 A1
- US 20070131678 A1
- US 20050082283 A1
- JP 4603932 B
- US 20110132902 A1